# EUROPEAN PATENT SPECIFICATION

(11) **EP 2 083 773 B1**
(45) Date of publication and mention of the grant of the patent: **21.08.2013**
(21) Application number: 07839628.0
(22) Date of filing: 17.10.2007
(51) Int. Cl.: A61F 7/00, A61N 5/06

(54) **WOUND CARE SYSTEM WITH VACUUM-LIGHT THERAPY**
WUNDPFLEGESYSTEM MIT VAKUUMLICHTTHERAPIE
SYSTEME DE TRAITEMENT DES PLAIES AVEC PHOTOTHERAPIE SOUS VIDE

(30) Priority: 19.10.2006 US 852803 P
(43) Date of publication of application: 05.08.2009
(73) Proprietor: Thermotek, Inc., Flower Mound, TX 75028 (US)
(72) Inventor: QUISENBERRY, Tony, Highland Village, TX 75077 (US); BALACHANDRAN, Niran, Lewisville, TX 75077 (US)
(74) Representative: Stump, Beat
(86) International application number: PCT/US2007/022148
(87) International publication number: WO 2008/051417

(56) References cited:
- WO-A1-01/14012
- US-A1- 2004 193 218
- US-A1- 2005 143 797
- US-A1- 2006 058 714

## Description

### BACKGROUND OF THE INVENTION

### Technical Field

The present invention relates to a wound care system with vacuum-light therapy; and more particularly, but not by way of limitation, to a programmable wound care control unit configured to generate a negative pressure for wound cleaning with light therapy, and, in one embodiment, oxygenation of a wound area for healing in conjunction with high thermal contrast modalities generated by the control unit.

### Description of the Related Art

US 2004/0193218 discloses a system for wound care with a cannula to be placed into the wound and fixed with a bandage. The system disclosed allows for less numerous dressing changes and therefore reduces the costs and efforts for such dressing changes.

WO 01/14012 discloses a system for light therapy of the human body. The system disposed allows for light therapy by means of LED's integrated in a pad, which in turn is adapted for contact with the skin. Further, cooling means integrated in the pad are disclosed in order to dissipate the heat generated by the LED's in operation, such that such heat cannot reach the patent's skin.
It is an object of the present invention provide for light therapy of wounds which can be combined with further therapy.
This object is solved by a wound care system according to claim 1. As the system for treatment of the wound area takes effect as a dressing and is easy to apply and to change, wounds can be treated.

### BRIEF SUMMARY OF THE INVENTION

The present invention relates to a wound care system with vacuum-light therapy. More particularly, one embodiment of the present invention includes a control unit for generating a negative pressure for wound cleaning with light therapy. The control unit may also be configured to deliver oxygen to a wound area in conjunction with high thermal contrast modalities. The invention is as defined in the appended claims.

### BRIEF DESCRIPTION OF THE DRAWINGS

A more complete understanding of the apparatus of the present invention may be obtained by reference to the following Detailed Description when taken in conjunction with the accompanying Drawings wherein:

FIG. 1 is an illustration of the wound care system according to an embodiment of the present invention;

FIG. 2 is a block diagram according to an embodiment of the present invention;

FIG. 3 is a flow diagram of a process according to an embodiment of the present invention;

FIG. 4 illustrates a side elevational cross sectional view of a therapy blanket/pad according to an embodiment of the present invention;

FIG. 5 illustrates a side elevational cross sectional view of a therapy blanket/pad according to an alternate embodiment of the present invention;

FIG. 6 is a diagrammatic illustration of a therapy blanket/pad according to an embodiment of the present invention; and

FIG. 7 is a diagrammatic illustration of a wound evacuation and UV LED treatment pad according to an embodiment of the present invention.

### DETAILED DESCRIPTION OF THE INVENTION

Various embodiments of the present invention will now be described more fully with reference to the accompanying drawings. The invention may, however, be embodied in many different forms and should not be constructed as limited to the embodiments set forth herein; rather, the embodiments are provided so that this disclosure will be thorough and complete, and will fully convey the scope of the invention to those skilled in the art.

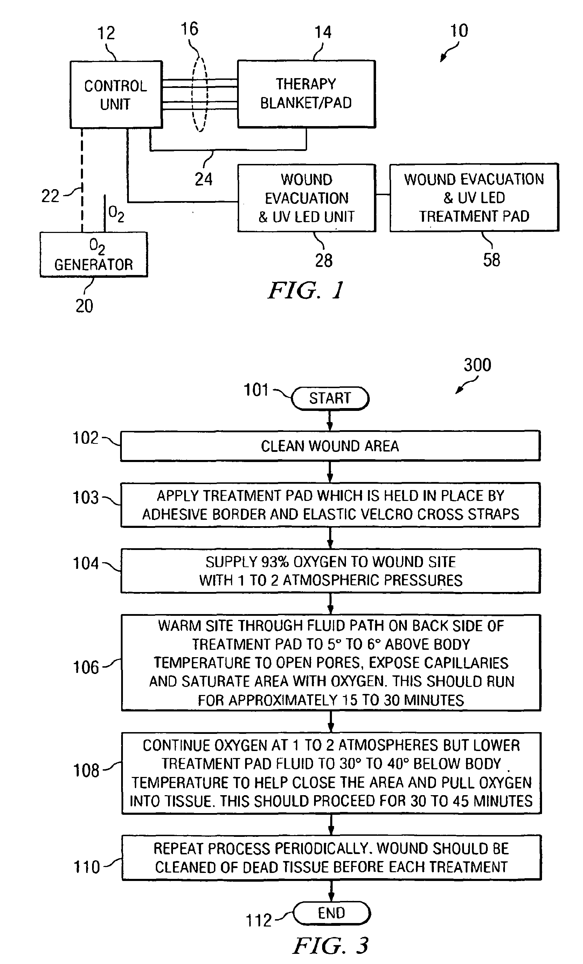

Referring first to FIGURE 1, there is shown an illustration of one embodiment of a wound care system 10 in accordance with principles of the present invention. The system 10 comprises a control unit 12, a therapy blanket/pad 14 and a plurality of tubular members 16 (to be defined below) connecting the control unit 12 to the therapy blanket/pad 14. The system 10 further includes a wound evacuation and ultra violet light emitting diode (UV LED) unit 28 and a wound evacuation and UV LED treatment pad 58. The wound evacuation and UV LED unit 28 is connected to the control unit 12 while the wound evacuation and UV LED treatment pad 58 is connected to the wound evacuation and UV LED unit 28. A system for providing both oxygenation therapy in conjunction with certain aspects of thermal therapy and fully describing the thermal operation and sequence compression aspects of one embodiment of the present invention is set forth and shown in U.S. Patent Application No 10/894,369, assigned to the assignee of the present invention. For that reason, thermal detail relative to the interaction between the control unit 12 and the therapy blanket/pad 14 relative to the thermal fluid flow and pressurization for sequenced compression therapy is not further defined herein. What is defined, is the added aspect of wound care provided by wound evacuation and light therapy. Light therapy is the application of light energy to the skin for therapeutic benefits. LED light therapy promotes wound healing and human tissue growth. Energy delivered by the LEDs enhances cellular metabolism, accelerates the repair and replenishment of damaged skin cells, as well as stimulates the production of collagen which is the foundation of a healthy and smooth skin. Light therapy is non-ablative, non-invasive, and painless.

Still referring to FIGURE 1, the use of the therapy blanket/pad 14 to the wound site of the patient may be, in one embodiment, subsequent to the cleaning of the wound area of dead tissue by the wound evacuation and UV LED treatment pad 58. In one embodiment, Velcro cross straps may be utilized to secure the therapy blanket/pad 14. A 93% concentration of oxygen has been suggested to be advantageous when applied to a wound site as described herein with one or two atmospheres of pressure. In accordance with one aspect of the present invention, an oxygen generator/concentrator 20 may be utilized within the control unit 12 or may be separate therefrom. In FIGURE 1, an oxygen generator/ concentrator 20 is shown in association with the control unit 12 by dotted line 22 and an oxygenation gas line 24 shown extending between the control unit 12 and the therapy blanket/pad 14 as a diagrammatic illustration according to an embodiment of the present invention.

In FIGURE 1, fiber optic strands (not explicitly shown) direct ultraviolet light from a plurality ofLEDs (not explicitly shown) to an array of fiber optic strand ends (not explicitly shown) located on the undersurface of wound evacuation and UV LED treatment pad 58. The control unit 12 may be used to modulate the ultraviolet light to create various patterns of light, different intensities of light, and different durations of light. The ultraviolet light is capable of penetrating through several layers of skin to destroy infectious bacteria. In one embodiment, not specifically shown herein, the UV LED treatment pad 58 may be provided on the therapy blanket/pad 14. According to exemplary embodiments, the ultraviolet light from the plurality of LEDs located on the undersurface of wound evacuation and UV LED treatment pad 58 destroys a wide variety of microorganisms such as, for example, bacteria which causes skin infections. In addition, the ultraviolet light from the plurality ofLEDs improves wound healing along with cell and bone growth. Furthermore, the use ofLEDs in light therapy is safe, non-invasive, drug-free and therapeutic.

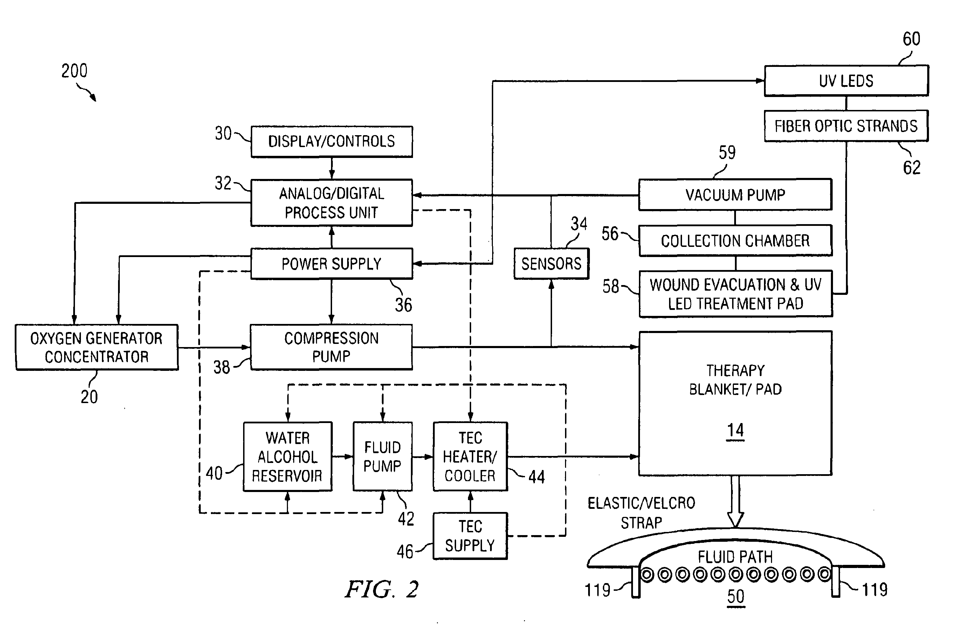

Referring now to FIGURE 2, there is a block diagram 200 illustrating the flow of oxygenation gas as a transfer fluid according to an embodiment of the present invention. As set forth in the block diagram 200, a control unit display 30 is provided in conjunction with an analog/digital processing unit 32. The process is referred to herein as "oxytherm" which is the term currently being utilized by the Applicant of the present invention through its assignee in preparation for commercial disclosure of certain ones of the methods, systems and principles of the present invention.

Still referring to FIGURE 2, a plurality of sensors 34 are utilized in conjunction with the processing unit 32 for control of heat transfer fluids to the therapy blanket/pad 14 as well as the oxygen delivery thereto. The oxygen generator/concentrator 20 is connected to a power supply 36, which power supply 36, also powers the processing unit 32. The oxygen generated from the oxygen generator/concentrator 20 is then pumped through compression pump 38 before delivery to the therapy blanket/pad 14. It should be noted that an oxygen supply may also be used.

Referring still to FIGURE 2, a water/alcohol reservoir 40 is shown in fluid flow communication with fluid pump 42 and Thermo Electric Cooler (TEC) heater/cooler 44. The TEC heater/cooler 44 is controlled by the processing unit 32 and a TEC supply 46 is likewise shown. Adjacent the TEC supply 46 is illustrated a diagrammatical schematic of a treatment chamber 50 defined beneath the therapy blanket/pad 14 wherein the treatment chamber 50 is thermally exposed to the thermal fluid by the fluid path therein illustrated. The adhesive attachment edges 52 therein shown likewise define the treatment chamber space 50 between the therapy blanket/pad 14 and the wound site to allow for the flow of the oxygenation gas therein.

Referring still to FIGURE 2, there is shown a vacuum pump 59 powered by the power supply 36. A collection chamber 56 is connected to the vacuum pump 54 and to a wound evacuation and UV LED treatment pad 58. The wound evacuation and UV LED treatment pad 58 is used prior to the therapy blanket/pad 14, in one embodiment of the present invention, for cleaning the wound area in preparation for oxygenation in conjunction with thermal therapy in accordance with the present invention.

Referring still to FIGURE 2, there is shown a plurality of ultraviolet LEDs 60 and fiber optic strands 62, which are interoperably connected to the wound evacuation and UV LED treatment pad 58. The wound evacuation and UV LED treatment pad 58 is used prior to the therapy blanket/pad 14, in one embodiment of the present invention, for removing bacteria from the wound area in preparation for oxygenation in conjunction with thermal therapy in accordance with the present invention. According to exemplary embodiments, the ultraviolet light from the plurality of LEDs located on the undersurface of wound evacuation and UV LED treatment pad 58 destroys a wide variety of microorganisms such as, for example, bacteria which causes skin infections. In addition, the ultraviolet light from the plurality of LEDs improves wound healing along with cell and bone growth. Furthermore, the use of LEDs in light therapy is safe, non-invasive, drug-free and therapeutic.

According to exemplary embodiments, the ultraviolet light from the plurality ofLEDs located on the undersurface of wound evacuation and UV LED treatment pad 58 is in the range of approximately 200 to 450 nanometers and higher, and energy levels of up to 35,000 microwatt seconds/cm², which are necessary to eliminate or destroy most microorganisms such as bacteria, spores, algae and viruses. Most bacteria can be destroyed at ultra violet energies of from about 3,000 to about 5,000 microwatt-seconds/cm² while mold spores may require energies in the 20,000 to 35,000 mW-seconds/cm².

Referring now to FIGURE 3 there is shown a flow diagram of a process 300 according to an embodiment of the present invention. The process 300 starts at step 101. At step 102, the wound area is cleaned of dead tissue, any undesirable fluids, and bacteria by applying the wound evacuation and UV LED treatment pad 58. The wound evacuation and UV LED treatment pad 58 is used prior to the therapy blanket/pad 14 for removing bacteria from the wound area in preparation for oxygenation in conjunction with thermal therapy in accordance with the present invention. According to exemplary embodiments, the ultraviolet light from the plurality of LEDs located on the undersurface of wound evacuation and UV LED treatment pad 58 destroys a wide variety of microorganisms such as, for example, bacteria which causes skin infections. In addition, the ultraviolet light from the plurality of LEDs improves wound healing along with cell and bone growth. Furthermore, the use of LEDs in light therapy is safe, non-invasive, drug-free and therapeutic.

At step 103, the therapy blanket/pad 14 is applied to the wound area. The therapy blanket/pad 14 is held in position by an adhesive border and, in one embodiment, elastic Velcro cross straps. At step 104, according to an embodiment, an oxygenation gas comprising on the order of 93% concentration of oxygen gas is delivered to the wound site with one to two atmospheric pressures. The numbers as set forth and shown are exemplary and other oxygenation concentrations as well as pressures are contemplated in accordance with the principles of the present invention. Consistent therewith, however, is the concept of, and teachings for, thermal treatment of the wound site in conjunction with the oxygenation. In accordance therewith, the site is thus warmed through the fluid path herein shown on the back side of the therapy blanket/pad 14 up to 5 to 6 degrees above the body temperature of the patient in step 106. This warming allows the pore of the patient's skin to open and expose capillaries therein. The capillaries of the skin are then saturated with oxygen. In one period of time herein described, a period of 15 to 30 minutes is recommended. At step 108, oxygenation is continued at one to two atmospheres and the therapy blanket/pad fluid is lowered to 30 to 40 degrees below body temperatures to help close the pores of the area and pull oxygen into the underlying tissue. This step then proceeds for approximately for 30 to 45 minutes in accordance with an embodiment of the present invention. At step 110, the process 300 may be repeated periodically and the wound site may be cleaned of dead tissue before each treatment. At step 112, the process 300 ends.

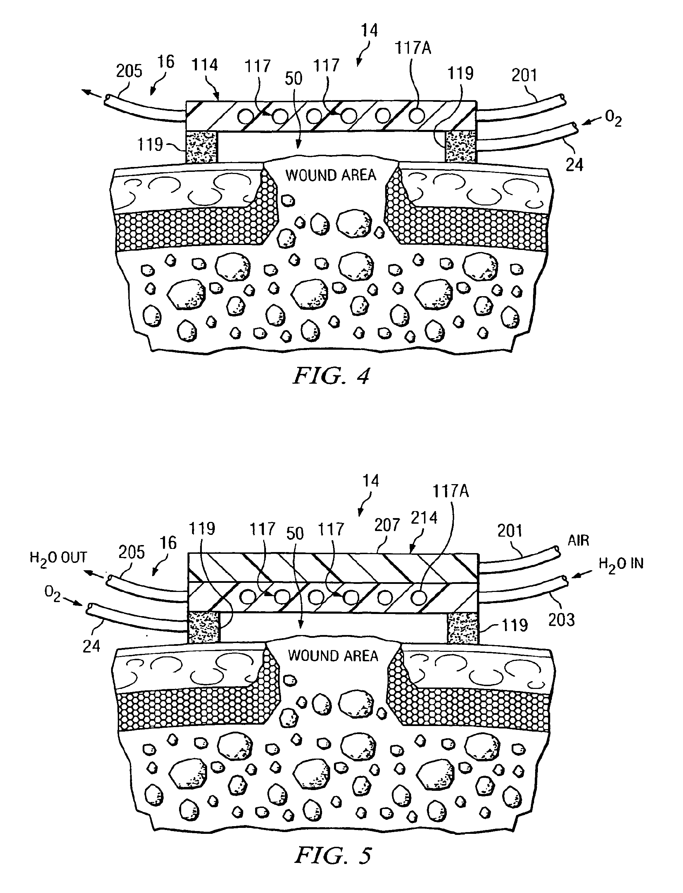

FIGURE 4 is a side elevational, cross sectional view of one embodiment of the therapy blanket/pad 14 of the present invention. In this embodiment, the therapy blanket/pad 14 is constructed within a single bladder configuration 114 where thermal fluid flow may be provided. The tubes 16 are coupled to the therapy blanket/pad 14, which is fabricated with a circuitous flow path therein for thermal fluid flow. This path may be tubular in form, or simply a path within therapy blanket/pad 14 defined by flow channels. What is shown is a path 117 within therapy blanket/pad 14. The path 117 is shown with tubular ends 117A, for example, in order to illustrate that thermal fluid flows therein for thermal treatment of the underlying wound area. Again, the path 117 may not be of tubular form and may have a variety of shapes and fabrication techniques well know in the art of thermal pads.

According to an exemplary embodiment, the therapy blanket/pad 14 is separated from the patient's skin by adhesive strips 119 having a thickness of, for example, 1/8 inch. The therapy blanket/pad 14 (not drawn to scale) exposes the wound to heat and then cold via path 117 while oxygen is injected into chamber 50. The injection of oxygen in conjunction with the aforesaid heating and cooling via path 117 helps treat the wound and any stasis zones therein where tissue swelling has restricted the flow of blood to the wound tissues. It is well known that without sufficient blood flow the epithelial and subcutaneous tissues referenced above receive less oxygen and are less able to migrate over the wound to promote heating. By utilizing the methods and apparatus of the present invention, oxygenation is enhanced and the problems associated with such conditions mitigated.

FIGURE 5 illustrates an alternative embodiment of the thermal therapy and oxygenation treatment pad of FIGURE 4. A dual bladder configuration 214 is thus provided where air may be applied to bladder 207 atop the thermal flow tubes 117, also represented by the "tubular" ends 117A shown for purposes of example only. In this manner, select compression therapy may be afforded in conjunction with the thermal and oxygenation treatment. In that regard, air inlet tube 201 is illustrated in connection to bladder 207. Both FIGURES 4 and 5 show oxygen tube 24 for feeding oxygen to chamber 50, with tube 203 allowing thermal fluid into conduits 117 with tube 205 allowing thermal fluid return to control unit 12 of FIGURE 1. FIGURE 5 further illustrates the advantages of FIGURE 4 with the ability for either compression or sequenced compression as referenced above.

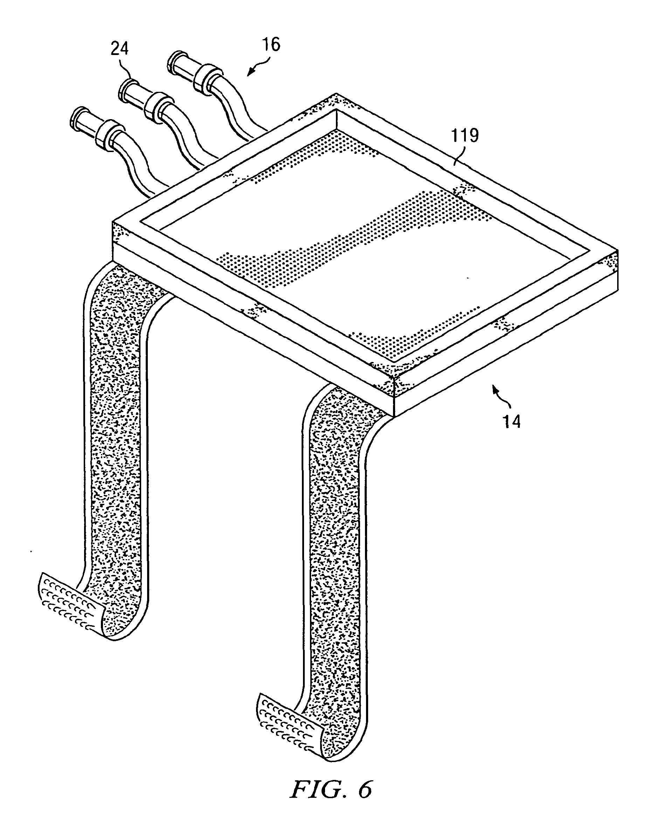

Referring now to FIGURE 6, there is shown a diagrammatic illustration of a therapy blanket/pad 14 of FIGURES 1 and 4. The tubular connections 16 for thermal fluid flow and the tube 24 for oxygen flow is clearly seen. The adhesive border 119 is likewise shown, as further outlined for clarity in the photograph of the prototype shown herein..

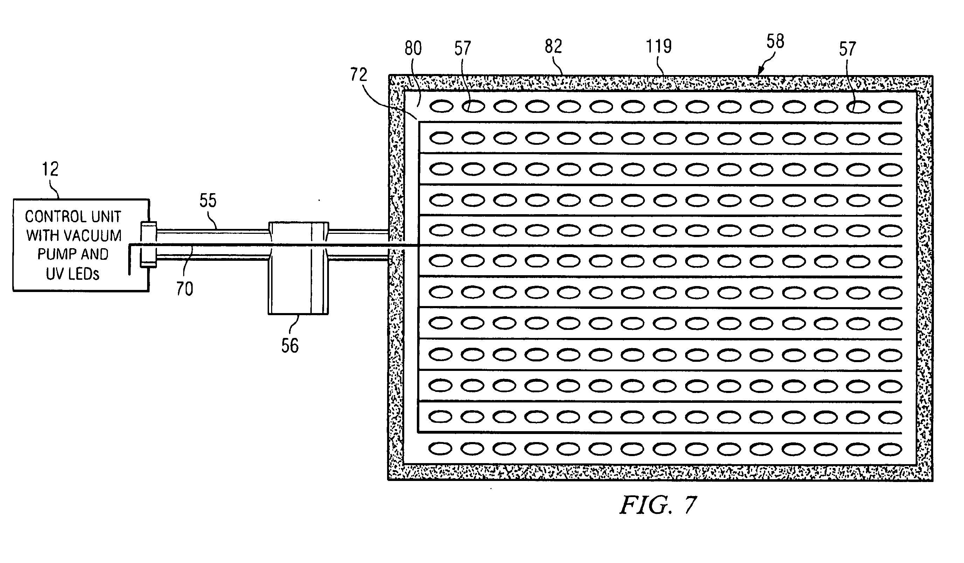

FIGURE 7 is diagrammatic illustration of a wound evacuation and UV LED treatment pad 58 according to an embodiment of the present invention. In this embodiment, the wound evacuation and UV LED treatment pad 58 contains an array of fiber optic strand ends 72 to project ultraviolet light onto a wound area (not explicitly shown). The wound evacuation and UV LED treatment pad 58 also contains an array of unique removal ports 57 that may be used to remove any undesirable fluid from the wound area. The wound evacuation and UV LED treatment pad 58 further contains a non-tissue adhesive service 80 which contains both the fiber optic strand array 72 and the unique removal ports 57. An adhesive circumference 82 is located around the periphery of the wound evacuation and UV LED treatment pad 58 to allow for a seal to be formed around the wound area. The seal, in conjunction with the removal ports 57, allows for a negative pressure to form over the wound area, which facilitates the removal the undesirable tissues from the wound area. The device also includes a control unit 12, which contains a vacuum pump (not shown) and a plurality of ultraviolet LEDs (not explicitly shown). The vacuum pump is connected to the wound evacuation and UV LED treatment pad 58 via a vacuum line 55. A collection chamber 56 is positioned inline between the vacuum pump and the wound evacuation and UV LED treatment pad 58 to intercept and store any undesirable fluids or the like that are removed from the wound area as a result of applying a negative pressure to the wound area with the vacuum pump. The plurality of ultraviolet LEDs transmit ultraviolet light through fiber optic strands 70 to the wound evacuation and UV LED treatment pad 58, where the strands are then dispersed throughout the wound evacuation and UV LED treatment pad 58 to project ultraviolet light onto the wound area. Energy delivered by the plurality of LEDs enhances cellular metabolism, accelerates the repair and replenishment of damaged skin cells, as well as stimulates the production of collagen which is the foundation of a healthy and smooth skin. Light therapy is non-ablative, non-invasive, and painless.

The previous Detailed Description is of embodiment(s) of the invention. The scope of the invention should not necessarily be limited by this Description. The scope of the invention is instead defined by the following claims.

## Claims

1. A system for treatment of a wound area of a patient comprising removal parts for exposing the wound area to a negative pressure, a control unit (12) and a plurality of Light Emitting Diodes (LEDs), **characterized by**:
a first treatment pad comprising the LED's being for cleaning and exposing a wound area to ultraviolet light;
a second treatment pad (58) comprising the removal ports for exposing the wound area to a negative pressure;
an adhesive strip (119) arranged on the second treatment pad and located around the periphery of fhe treatment pad such as to separate the treatment pads from the patient's skin and to form a treatment chamber (50) for the wound area
whereby the control unit (12) is interoperably connected to the first treatment pad and the second treatment pad (58) for providing a negative pressure and the ultraviolet light to the wound area.

2. The system of claim 1, wherein said plurality of LEDs are located on an underside of the first treatment pad.

3. The system of claim 1, wherein said control unit (12) is adapted to control the intensity of said ultraviolet light emitted from the plurality of LEDs.

4. The system of claim 1, wherein said control unit (12) is adapted to control the duration of said ultraviolet light emitted from the plurality of LEDs.

5. The system of claim 1, wherein said second therapy pad is adapted to oxygenate said wound area with oxygen rich air and expose said wound area to thermal differentials.

6. The system of claim 1, wherein said first treatment pad provides said ultraviolet light to the wound area prior to second therapy pad providing oxygenation and thermal exposure.

7. The system of claim 1, wherein said second therapy pad provides heating therapy to a patient during oxygenation.

8. The system of claim 1, wherein said second therapy pad provides cooling therapy to a patient after a select period of time in which oxygenation has occurred at said wound area.

9. The system of claim 1, wherein said second therapy pad provides contrast therapy to a patient in conjunction with oxygenation, wherein said wound area is heated before oxygenation and cooled thereafter.

10. The system of claim 1, wherein said second therapy pad further comprises: an inlet port for receiving heat transfer fluid from the control unit (12); an outlet port for returning the heat transfer fluid to the control unit (12); and means for providing oxygenation at select pressures and time at said wound area.

## Patentansprüche

1. System zum Behandeln eines Wundgebietes eines Patienten, das mehrere Anschlüsse um das Wundgebiet einem negativen Druck auszusetzen, eine Steuerungseinheit (12) und mehrere Licht emittierende Dioden (LEDs) aufweist, **gekennzeichnet durch** ein erstes Behandlungskissen, das die LEDs zum Reinigen und Bestrahlen eines Wundgebietes mit Ultraviolettlicht umfasst; ein zweites Behandlungskissen (58), das die Anschlüsse aufweist, um das Wundgebiet einem negativen Druck auszusetzen; einen Adhäsivstreifen (119), der auf dem zweiten Behandlungskissen angeordnet ist und sich der Peripherie des Behandlungskissens entlang erstreckt, um die Behandlungskissen von der Haut des Patienten zu trennen und eine Behandlungskammer (50) für das Wundgebiet zu bilden, wobei die Steuerungseinheit (12) betriebsfähig mit dem erste Behandlungskissen und dem zweiten Behandlungskissen (58)verbunden ist, um im Wundgebiet negativem Druck und Ultraviolettlicht bereitzustellen.

2. System nach Anspruch 1, wobei sich die mehreren LEDs an einer Unterseite des ersten Behandlungskissens befinden.

3. System nach Anspruch 1, wobei die Steuerungseinheit (12) dafür ausgelegt ist, die Stärke des Ultraviolettlichtes zu steuern, das von den mehreren LEDs emittiert wird.

4. System nach Anspruch 1, wobei die Steuerungseinheit (12) dafür ausgelegt ist, die Dauer des Ultraviolettlichtes zu steuern, das von den mehreren LEDs emittiert wird.

5. System nach Anspruch 1, wobei das zweite Therapiekissen dafür ausgelegt ist, das Wundgebiet durch sauerstoffreiche Luft mit Sauerstoff anzureichern und das Wundgebiet Temperaturdifferenzen auszusetzen.

6. System nach Anspruch 1, wobei das erste Behandlungskissen das Ultraviolettlicht für das Wundgebiet bereitstellt, bevor das zweite Therapiekissen die Sauerstoffanreicherung und die thermische Exposition bereitstellt.

7. System nach Anspruch 1, wobei das zweite Therapiekissen während der Sauerstoffanreicherung eine Erwärmungstherapie für einen Patienten bereitstellt.

8. System nach Anspruch 1, wobei das zweite Therapiekissen nach einer ausgewählten Zeitdauer, nachdem die Sauerstoffanreicherung des Wundgebietes stattgefunden hat, eine Kühltherapie für einen Patienten bereitstellt.

9. System nach Anspruch 1, wobei das zweite Therapiekissen für einen Patienten eine Kontrasttherapie in Verbindung mit einer Sauerstoffanreicherung bereitstellt, wobei das Wundgebiet vor der Sauerstoffanreicherung erwärmt und danach abgekühlt wird.

10. System nach Anspruch 1, wobei das zweite Therapiekissen weiter umfasst: einen Eingang zum Aufnehmen des Wärmeübertragungsfluids aus der Steuerungseinheit (12); eine Auslass zur Rückführung des Wärmeübertragungsfluids zur Steuerungseinheit (12); und Mittel zum Erzielen einer Sauerstoffanreicherung an dem Wundgebiet bei ausgewählten Drücken und für eine ausgewählte Dauer.

## Revendications

1. Système de traitement d'une plaie d'un patient, comprenant des ports de retrait pour exposer la plaie à une pression négative, une unité de commande (12) et une pluralité de diodes électroluminescentes (LED), **caractérisé par** :
un premier accessoire de traitement comprenant les LED et destiné à nettoyer et exposer une plaie à une lumière ultraviolette ;
un second accessoire de traitement (58) comprenant les ports de retrait pour exposer la plaie à une pression négative ;
une bande adhésive (119) disposée sur le second accessoire de traitement et située sur la périphérie de l'accessoire de traitement de manière à séparer les accessoires de traitement de la peau du patient et à former une cellule de traitement (50) pour la plaie,
l'unité de commande (12) étant interconnectée opérationnellement au premier accessoire de traitement et au second accessoire de traitement (58) pour fournir une pression négative et la lumière ultraviolette à la plaie.

2. Système selon la revendication 1, dans lequel ladite pluralité de LED sont situées sur une face inférieure du premier accessoire de traitement.

3. Système selon la revendication 1, dans lequel ladite unité de commande (12) est apte à commander l'intensité de ladite lumière ultraviolette émise par la pluralité de LED.

4. Système selon la revendication 1, dans lequel ladite unité de commande (12) est apte à commander la durée de ladite lumière ultraviolette émise par la pluralité de LED.

5. Système selon la revendication 1, dans lequel ledit second accessoire thérapeutique est apte à oxygéner ladite plaie avec de l'air riche en oxygène et à exposer ladite plaie à des différentiels thermiques.

6. Système selon la revendication 1, dans lequel ledit premier accessoire de traitement fournit ladite lumière ultraviolette à la plaie avant que le second accessoire de traitement assure l'oxygénation et une exposition thermique.

7. Système selon la revendication 1, dans lequel ledit second accessoire thérapeutique dispense une thérapie thermique à un patient pendant l'oxygénation.

8. Système selon la revendication 1, dans lequel ledit second accessoire thérapeutique dispense une thérapie réfrigérante à un patient pendant un laps de temps pendant lequel une oxygénation a eu lieu au niveau de ladite plaie.

9. Système selon la revendication 1, dans lequel ledit second accessoire thérapeutique dispense une thérapie contrastée en conjonction avec l'oxygénation, ladite plaie étant chauffée avant oxygénation puis refroidie.

10. Système selon la revendication 1, dans lequel ledit second accessoire thérapeutique comprend en outre : un port d'admission pour recevoir du fluide de transfert thermique de l'unité de commande (12) ; un port de sortie pour renvoyer le fluide de transfert thermique vers l'unité de commande (12) ; et un moyen pour assurer une oxygénation sous des pressions et à des moments sélectionnés au niveau de ladite plaie.
